# EUROPÄISCHE PATENTSCHRIFT

(11) **EP 3 999 015 B1**
(45) Veröffentlichungstag und Bekanntmachung des Hinweises auf die Patenterteilung: **09.09.2026**
(21) Anmeldenummer: 20737426.5
(22) Anmeldetag: 06.07.2020
(51) Int. Cl.: A61K 8/02, A61K 8/26, A61K 8/58, A61Q 5/06

(54) **BESCHICHTETE EFFEKTPIGMENTE UND DEREN HERSTELLUNG**
COATED EFFECT PIGMENTS AND PRODUCTION THEREOF
PIGMENTS À EFFET ENROBÉS ET LEUR FABRICATION

(30) Priorität: 19.07.2019 DE 102019210694
(43) Veröffentlichungstag der Anmeldung: 25.05.2022
(73) Patentinhaber: Henkel AG & Co. KGaA, 40589 Düsseldorf (DE)
(72) Erfinder: LECHNER, Torsten, 40764 Langenfeld (DE); WESER, Gabriele, 41472 Neuss (DE); KRIENER, Caroline, 40229 Düsseldorf (DE); KOLONKO, Claudia, 42857 Remscheid (DE); SCHUMACHER, Ulrike, 40589 Düsseldorf (DE); NOWOTTNY, Marc, 41069 Mönchengladbach (DE); SCHOEPGENS, Juergen, 41366 Schwalmtal (DE); JAISER, Phillip, 40764 Langenfeld (DE); MATHIASZYK, Carsten, 45277 Essen (DE); HODES, Jing, 58093 Hagen (DE)
(86) Internationale Anmeldenummer: PCT/EP2020/068957
(87) Internationale Veröffentlichungsnummer: WO 2021/013510

(56) Entgegenhaltungen:
- EP-A1- 2 902 451
- DE-A1- 102011 055 072
- DE-A1- 102012 000 887

## Beschreibung

Gegenstand der vorliegenden Anmeldung sind Effektpigmente, die ein Substratplättchen und eine Beschichtung umfassen, wobei die Beschichtung mindestens eine Schicht aufweist, die ein Metalloxid und/oder Metalloxidhydrat umfasst. Die Anmeldung beschreibt ferner ein Verfahren zur Herstellung der Effektpigmente.

Die Veränderung von Form und Farbe von keratinischen Fasern, insbesondere von Haaren, stellt einen wichtigen Bereich der modernen Kosmetik dar. Zur Veränderung der Haarfarbe kennt der Fachmann je nach Anforderung an die Färbung diverse Färbesysteme. Für permanente, intensive Färbungen mit guten Echtheitseigenschaften und guter Grauabdeckung werden üblicherweise Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch sehr langanhaltende Färbeergebnisse aus.

Bei dem Einsatz von direktziehenden Farbstoffen diffundieren bereits fertig ausgebildete Farbstoffe aus dem Färbemittel in die Haarfaser hinein. Im Vergleich zur oxidativen Haarfärbung weisen die mit direktziehenden Farbstoffen erhaltenen Färbungen eine geringere Haltbarkeit und schnellere Auswaschbarkeit auf. Färbungen mit direktziehenden Farbstoffen verbleiben üblicherweise für einen Zeitraum zwischen 5 und 20 Haarwäschen auf dem Haar.

Für kurzzeitige Farbveränderungen auf dem Haar und/oder der Haut ist der Einsatz von Farbpigmenten bekannt. Unter Farbpigmenten werden im Allgemeinen unlösliche, farbgebende Substanzen verstanden. Diese liegen ungelöst in Form kleiner Partikel in der Färbeformulierung vor und lagern sich lediglich von außen auf den Haarfasern und/oder der Hautoberfläche ab. Daher lassen sie sich in der Regel durch einige Wäschen mit Tensid-haltigen Reinigungsmitteln wieder rückstandslos entfernen. Unter dem Namen Haar-Mascara sind verschiedene Produkte dieses Typs auf dem Markt erhältlich.

Wünscht sich der Anwender besonders langanhaltende Färbungen, so ist die Verwendung von oxidativen Färbemitteln bislang seine einzige Option. Doch trotz vielfacher Optimierungsversuche lässt sich bei der oxidativen Haarfärbung ein unangenehmer Ammoniakgeruch bzw. Amingeruch nicht vollständig vermeiden. Auch die mit dem Einsatz der oxidativen Färbemittel nach wie vor verbundene Haarschädigung wirkt sich auf das Haar des Anwenders nachteilig aus.

EP 2168633 B1 beschäftigt sich mit der Aufgabenstellung, langanhaltende Haarfärbungen unter Einsatz von Pigmenten zu erzeugen. Die Schrift lehrt, dass sich bei Verwendung der Kombination aus einem Pigment, einer organischer Silicium-Verbindung, einem filmbildenden Polymer und einem Lösungsmittel auf Haaren Färbungen erzeugen lassen, die gegenüber Shampoonierungen besonders widerstandsfähig sind.

Metallische Glanzpigmente oder Metalleffektpigmente finden breite Anwendung in vielen Bereichen der Technik. Sie werden beispielsweise zur Einfärbung von Lacken, Druckfarben, Tinten, Kunststoffen, Gläsern keramischen Produkten und Zubereitungen der dekorativen Kosmetik wie Nagellack eingesetzt. Sie zeichnen sich vor allem durch ihren reizvollen winkelabhängigen Farbeindruck (Goniochromie) und ihren metallartig wirkenden Glanz aus.

Aus der DE 10 2012 000887 A1 sind Effektpigment bekannt, welche auf einem plättchenförmigen Substrat basieren, wobei das Substrat mit mindestens einer hochbrechenden Schicht beschichtet ist.

Die EP 2 902 451 A1 beschreibt ein Verfahren zum Beschichten von Metalleffektpigmenten. In diesem Verfahren werden Substratplättchen nass-chemisch unter Verwendung eines Siliciumalkoxids beschichtet.

DE 10 2011 055072 A1 offenbart nasschemisch oxidierte Aluminiumeffektpigmente, die eine von Aluminiumoxid verschiedene Metalloxidschicht und eine umhüllende organische Polymer-schicht aufweisen.

Haare mit einem Metallic Finish oder metallischen Reflexen sind im Trend. Durch den Metallic-Ton wirkt das Haar dicker und glänzender.

Es besteht Bedarf, Effektpigmente, insbesondere für Haarfärbungen, bereitzustellen, die einerseits eine hohe Wasch- und Reibechtheit und andererseits die Haareigenschaften wie Handhabbarkeit und Haptik nicht negativ beeinträchtigen. Dazu wäre es wünschenswert, wenn die eingesetzten Effektpigmente eine hohe Deckkraft aufwiesen und in dünnen Schichten auf dem Haar aufgebracht werden könnten.

Die Effektpigmente sollten insbesondere für Färbesysteme geeignet sein, die ohne den Einsatz von Oxidationsmitteln und/oder Oxidationsfarbstoffvorprodukte auskommen.

Überraschenderweise hat sich nun herausgestellt, dass die vorgenannten Aufgaben hervorragend gelöst werden können durch ein Effektpigmentmit den Merkmalen des Anspruchs 1.

Es hat sich gezeigt, dass Haarfärbungen mit solchen Effektpigmenten eine hohe Wasch- und Reibechtheit aufweisen.

Das Effektpigment weist ein Substratplättchen auf.

Das Substratplättchen weist vorzugsweise eine durchschnittliche Dicke von höchstens 150 nm, vorzugsweise weniger als 50 nm, mehr bevorzugt weniger als 30 nm, besonders bevorzugt höchstens 25 nm, beispielsweise höchstens 20 nm auf. Die durchschnittliche Dicke der Substratplättchen beträgt mindestens 1 nm, vorzugsweise mindestens 2,5 nm, besonders bevorzugt mindestens 5 nm, beispielsweise mindestens 10 nm. Bevorzugte Bereiche für die Dicke des Substratplättchens sind 2,5 bis 50 nm, 5 bis 50 nm, 10 bis 50 nm; 2,5 bis 30 nm, 5 bis 30 nm, 10 bis 30 nm; 2,5 bis 25 nm, 5 bis 25 nm, 10 bis 25 nm, 2,5 bis 20 nm, 5 bis 20 nm und 10 bis 20 nm. Vorzugsweise weist jedes Substratplättchen eine möglichst einheitliche Dicke auf.

Das Substratplättchen ist vorzugsweise monolithisch aufgebaut. Monolithisch bedeutet in diesem Zusammenhang aus einer einzigen abgeschlossenen Einheit ohne Brüche, Schichtungen oder Einschlüsse bestehend, wobei jedoch innerhalb des Substratplättchens Gefügewechsel auftreten können. Das Substratplättchen ist vorzugsweise homogen aufgebaut, d.h. dass innerhalb des Plättchens kein Konzentrationsgradient auftritt. Insbesondere ist das Substratplättchen nicht schichtartig aufgebaut und weist keine darin verteilten Teilchen oder Partikel auf.

Die Größe des Substratplättchens kann auf den jeweiligen Anwendungszweck, beispielsweise dem gewünschten Effekt auf einem keratinischen Material, abgestimmt werden. In der Regel haben die Substratplättchen einen mittleren größten Durchmesser von etwa 2 bis 200 µm, insbesondere etwa 5 bis 100 µm.

In einer bevorzugten Ausführungsform beträgt der Formfaktor (Aspect Ratio), ausgedrückt durch das Verhältnis der mittleren Größe zur durchschnittlichen Dicke, mindestens 80, vorzugsweise mindestens 200, mehr bevorzugt mindestens 500, besonders bevorzugt mehr als 750. Dabei wird als mittlere Größe der unbeschichteten Substratplättchen der d50-Wert der unbeschichteten Substratplättchen verstanden. Der d50-Wert wurde, soweit nicht anders angegeben, mit einem Gerät des Typs Sympatec Helos mit Quixel-Nassdispergierung bestimmt. Dabei wurde zur Probenvorbereitung die zu untersuchende Probe für eine Dauer von 3 Minuten in Isopropanol vordispergiert.

Die Substratplättchen sind aus einem Metall oder einer Legierung aufgebaut.

Als Metall kommt jedes, für Effektpigmente geeignete Metall in Betracht. Derartige Metalle sind unter anderem Eisen und Stahl, sowie alle luft- und wasserbeständigen (Halb)metalle wie beispielsweise Platin, Zinn, Zink, Chrom, Molybdän und Silicium, sowie deren Legierungen wie Aluminiumbronzen und Messing. Bevorzugte Metalle sind Aluminium, Kupfer, Silber und Gold. Bevorzugte Substratplättchen sind Aluminiumplättchen und Messingplättchen, wobei Substratplättchen aus Aluminium besonders bevorzugt sind.

Substratplättchen aus Aluminium können unter anderem durch Herausstanzen aus Aluminiumfolie oder nach gängigen Mahl- und Verdüsungstechniken hergestellt werden. So sind beispielsweise Aluminiumplättchen aus dem Hallverfahren, einem Nassmahlverfahren, erhältlich.

Andere Metallplättchen, beispielsweise aus Bronze, können in einem Trockenmahlverfahren wie dem Hametagverfahren erhalten werden.

Die Substratplättchen können verschiedene Formen aufweisen. Als Substratplättchen können beispielsweise lamellare oder lentikulare Metallplättchen oder auch sogenannte *vacuum metallized pigments* (VMP) verwendet werden. Lamellare Substratplättchen zeichnen sich durch einen unregelmäßig strukturierten Rand aus und werden aufgrund ihres Erscheinungsbildes auch als "cornflakes" bezeichnet. Lentikulare Sustratplättchen weisen einen im Wesentlichen regelmäßigen runden Rand auf und werden aufgrund ihres Erscheinungsbildes auch als "silverdollars" bezeichnet.

Die Substratplättchen aus Metall oder Metalllegierung können passiviert sein, beispielsweise durch Eloxieren (Oxidschicht) oder Chromatieren.

Durch eine Beschichtung können die Oberflächeneigenschaften und/oder optischen Eigenschaften des Effektpigments verändert sowie die mechanische und chemische Belastbarkeit der Effektpigmente erhöht werden. Es können beispielsweise lediglich die obere und/oder untere Seite des Substratplättchens beschichtet sein, wobei die Seitenflächen ausgespart sind. Vorzugsweise ist die gesamte Oberfläche der gegebenenfalls passivierten Substratplättchen, einschließlich der Seitenflächen, von der Schicht bedeckt. Die Substratplättchen sind vorzugsweise vollständig von der Beschichtung umhüllt.

Die Beschichtung kann aus einer oder aus mehreren Schichten bestehen. In einer bevorzugten Ausführungsform weist die Beschichtung lediglich eine Schicht A auf. In einer ebenfalls bevorzugten Ausführungsform weist die Beschichtung insgesamt mindestens zwei, vorzugsweise zwei oder drei, Schichten auf. Es kann bevorzugt sein, die Beschichtung zwei Schichten A und B aufweist, wobei die Schicht B von der Schicht A verschieden ist. Vorzugsweise befindet sich Schicht A zwischen der Schicht B und der Oberfläche des Substratplättchens. In noch einer bevorzugten Ausführungsform weist die Beschichtung drei Schichten A, B und C auf. In dieser Ausführungsform befindet sich zwischen der Schicht B und der Oberfläche des Substratplättchens die Schicht A und auf der Schicht B befindet sich eine Schicht C, die von der darunterliegenden Schicht B verschieden ist.

Die mindestens eine Schicht wird nass-chemisch unter Verwendung eines Metallalkoxids, ausgewählt ist aus der Gruppe bestehend aus Tetramethylorthosilikat, Tetraethylorthosilikat, Tetraisopropylorthosilikat und Mischungen daraus und einer ausgewählten siliciumorganischen Verbindung mit einer basischen Gruppe hergestellt. Die mindestens eine Schicht enthält ein Silicium(di)oxid und/oder Siliciumoxidhydrat.

Siliciumdioxid ist bevorzugt. Die Schicht A weist bevorzugt eine Dicke von 1 bis 100 nm, besonders bevorzugt 5 bis 50 nm, insbesondere bevorzugt 5 bis 20 nm, auf.

Die Schicht B, falls vorhanden, ist von der Schicht A verschieden und kann mindestens ein hochbrechendes Metalloxid enthalten. Hochbrechende Materialien weisen einen Brechungsindex von mindestens 1,9, bevorzugt mindestens 2,0 und besonders bevorzugt mindestens 2,4 auf. Vorzugsweise umfasst die Schicht B mindestens 95 Gew.-%, besonders bevorzugt mindestens 99 Gew.-% an hochbrechenden Metalloxid(en).

Enthält die Schicht B ein (hochbrechendes) Metalloxid, weist sie vorzugsweise eine Dicke von mindestens 50 nm auf. Vorzugsweise beträgt die Dicke von Schicht B nicht mehr als 400 nm, besonders bevorzugt höchstens 300 nm.

Für Schicht B geeignete hochbrechende Metalloxide sind beispielsweise selektiv lichtabsorbierende (d.h. farbige) Metalloxide, wie beispielsweise Eisen(III)oxid (α- und γ-Fe2O3, rot), Cobalt(II)oxid (blau), Chrom(III)oxid (grün),Titan(III)oxid (blau, liegt üblicherweise im Gemisch mit Titanoxynitriden und Titannitriden vor) und Vanadium(V)oxid (orange) sowie deren Gemische. Es eignen sich auch farblose hochbrechende Oxide wie Titandioxid und/oder Zirkonoxid.

Schicht B kann einen selektiv absorbierenden Farbstoff enthalten, vorzugsweise 0,001 bis 5 Gew.-%, besonders bevorzugt 0,01 bis 1 Gew.-%, jeweils bezogen auf die Gesamtmenge der Schicht B. Geeignet sind organische und anorganische Farbstoffe, die sich stabil in eine Metalloxidbeschichtung einbauen lassen. Farbstoffe im Sinne der vorliegenden Erfindung besitzen eine Löslichkeit in Wasser (760 mmHg) bei 25 °C von mehr als 0,5 g/L und sind daher nicht als Pigmente anzusehen.

Alternativ zu einem Metalloxid kann Schicht B eine Metallpartikelträgerschicht mit auf der Oberfläche der Metallpartikelträgerschicht aufgebrachten Metallpartikeln umfassen. In einer bevorzugten Ausführungsform bedecken die Metallpartikel direkt einen Teil der Metallpartikelträgerschicht. In dieser Ausführungsform weist das Effektpigment Bereiche auf, in denen sich keine Metallpartikel befinden, d.h. Bereiche, die nicht mit den Metallpartikeln bedeckt sind.

Die Metallpartikelträgerschicht eine Metallschicht und/oder eine Metalloxidschicht umfassen.

Wenn die Metallpartikelträgerschicht eine Metallschicht und eine Metalloxidschicht umfasst, ist die Anordnung dieser Schichten nicht limitiert.

Es ist bevorzugt, dass die Metallpartikelträgerschicht wenigstens eine Metallschicht umfasst. Es ferner bevorzugt, dass die Metallschicht ein Element ausgewählt aus Zinn (Sn), Palladium (Pd), Platin (Pt) und Gold (Au) aufweist.

Die Metallschicht kann beispielsweise durch Zugabe von Alkali zu einer das Metall enthaltenden Metallsalzlösung gebildet werden.

Enthält die Metallpartikelträgerschicht eine Metalloxidschicht, umfasst diese vorzugsweise kein Siliciumdioxid. Die Metalloxidschicht enthält vorzugsweise ein Oxid von mindestens einem Element, ausgewählt aus der Gruppe bestehend aus von Mg (Magnesium), Sn (Zinn), Zn (Zink), Co (Kobalt), Ni (Nickel), Fe (Eisen), Zr (Zirkonium), Ti (Titan) und Ce (Cer). Besonders bevorzugt enthält die Metallpartikelträgerschicht iii) in Form einer Metalloxidschicht ein Metalloxid von Sn, Zn, Ti und Ce.

Die Herstellung der Metallpartikelträgerschicht in Form einer Metalloxidschicht kann beispielsweise durch Hydrolyse eines Alkoxids eines Metalls, das das Metall des Metalloxids bildet, in einem Sol-Gel Verfahren erfolgen.

Die Dicke der Metallpartikelträgerschicht beträgt vorzugsweise nicht mehr als 30 nm.

Die Metallpartikel kann wenigstens ein Element umfassen, welches ausgewählt ist aus der Gruppe bestehend aus Aluminium (AI), Titan (Ti), Chrom (Cr), Eisen (Fe), Kobalt (Co), Nickel (Ni), Kupfer (Cu), Zink (Zn), Ruthenium (Ru), Rhodium (Rh), Palladium (Pd), Silber (Ag), Zinn (Sn), Platin (Pt), Gold (Au) und deren Legierungen. Es ist insbesondere bevorzugt, dass die Metallpartikel wenigstens ein Element ausgewählt aus Kupfer (Cu), Nickel (Ni) und Silber (Ag) aufweisen.

Der durchschnittliche Partikeldurchmesser der Metallpartikel beträgt vorzugsweise nicht mehr als 50 nm, mehr bevorzugt nicht mehr als 30 nm. Der Abstand zwischen den Metallpartikeln beträgt vorzugsweise nicht mehr als 10 nm.

Als Verfahren zur Bildung der Metallpartikel eignen sich Vakuumverdampfung, Sputtern, Chemical Vapor Deposition (CVD), stromloses Plattieren oder dergleichen. Von diesen Verfahren ist das stromlose Plattieren besonders bevorzugt.

Gemäß einer bevorzugten Ausführungsform weisen die Effektpigmente eine weitere Schicht C, umfassend ein Metalloxid(hydrat), die von der darunterliegenden Schicht B verschieden ist, auf. Geeignete Metalloxide sind beispielsweise Silicium(di)oxid, Siliciumoxidhydrat, Aluminiumoxid, Aluminiumoxidhydrat, Zinkoxid, Zinnoxid, Titandioxid, Zirkonoxid, Eisen(III)oxid und Chrom(III)oxid. Bevorzugt ist Siliciumdioxid.

Die Schicht C weist vorzugsweise eine Dicke von 10 bis 500 nm, besonders bevorzugt 50 bis 300 nm auf.

Die Beschichtung des Effektpigments weist mindestens eine Schicht auf, die nass-chemisch aus einem Metallalkoxid, ausgewählt ist aus der Gruppe bestehend aus Tetramethylorthosilikat, Tetraethylorthosilikat, Tetraisopropylorthosilikat und Mischungen daraus, und einer ausgewählten siliciumorganischen Verbindung mit einer basischen Gruppe hergestellt worden ist.

Die mindestens eine Schicht, die unter Verwendung eines Metallalkoxids und einer siliciumorganischen Verbindung mit einer basischen Gruppe hergestellt worden ist, kann Schicht A, B und/oder C sein. Im Fall, dass die Beschichtung lediglich eine Schicht A aufweist, ist Schicht A unter Verwendung eines Metallalkoxids, ausgewählt ist aus der Gruppe bestehend aus Tetramethylorthosilikat, Tetraethylorthosilikat, Tetraisopropylorthosilikat und Mischungen daraus, und einer ausgewählten siliciumorganischen Verbindung mit einer basischen Gruppe hergestellt worden.

Im Fall, dass die Beschichtung des Effektpigments zwei Schichten A und B aufweist, ist Schicht B unter Verwendung eines Metallalkoxids, , ausgewählt ist aus der Gruppe bestehend aus Tetramethylorthosilikat, Tetraethylorthosilikat, Tetraisopropylorthosilikat und Mischungen daraus, und einer ausgewählten siliciumorganischen Verbindung mit einer basischen Gruppe hergestellt worden.

Im Fall, dass die Beschichtung die Schichten A, B und C aufweist, ist Schicht C unter Verwendung eines Metallalkoxids, ausgewählt ist aus der Gruppe bestehend aus Tetramethylorthosilikat, Tetraethylorthosilikat, Tetraisopropylorthosilikat und Mischungen daraus, und einer ausgewählten siliciumorganischen Verbindung mit einer basischen Gruppe hergestellt worden.

Es ist insbesondere bevorzugt, dass das Effektpigment ein Substratplättchen aus Aluminium und eine Schicht A, umfassend Siliciumdioxid und die siliciumorganische Verbindung mit einer basischen Gruppe, aufweist. Weist das Effektpigment auf Basis eines Substratplättchens eine Schicht A und eine Schicht C auf, ist es bevorzugt, dass das Effektpigment ein Substratplättchen aus Aluminium und Schichten A und C, umfassend Siliciumdioxid, aufweist, wobei zur Herstellung der Schicht C ferner die siliciumorganische Verbindung mit einer basischen Gruppe verwendet wurde.

Es ist erfindungswesentlich, dass bei der Herstellung der mindestens eine Schicht eine ausgewählte siliciumorganischen Verbindung mit einer basischen Gruppe eingesetzt wird.

Diese organischen Siliciumverbindungen mit einer basischen Gruppe sind reaktive Verbindungen.

Organische Siliciumverbindungen, die alternativ auch als siliciumorganische Verbindungen bezeichnet werden, sind Verbindungen, die entweder eine direkte Silicium-Kohlenstoff-Bindung (Si-C) aufweisen oder in denen der Kohlenstoff über ein Sauerstoff-, Stickstoff- oder Schwefel-Atom an das Silicium-Atom geknüpft ist. Die erfindungsgemäßen organische Siliciumverbindungen sind Verbindungen, die eine bis drei Siliciumatome enthalten. Besonders bevorzugt enthalten die organische Siliciumverbindungen ein oder zwei Siliciumatome.

Die Bezeichnung Silan steht nach den IUPAC-Regeln für eine Stoffgruppe chemischer Verbindungen, die auf einem Silicium-Grundgerüst und Wasserstoff basieren. Bei organischen Silanen sind die WasserstoffAtome ganz oder teilweise durch organische Gruppen wie beispielsweise (substituierte) Alkylgruppen und/oder Alkoxygruppen ersetzt. In den organischen Silanen kann auch ein Teil der Wasserstoffatome durch Hydroxygruppen ersetzt sein.

Die organische Siliciumverbindung mit einer basischen Gruppe entspricht der Formel (I).

IDie organische Siliciumverbindung entspricht der Formel (I)

R₁R₂N-L-Si(OR₃)ₐ(R₄)_{b} (I),

wobei
- R₁, R₂ unabhängig voneinander für ein Wasserstoffatom oder eine C₁-C₆-Alkylgruppe stehen,
- L für eine lineare oder verzweigte, zweiwertige C₁-C₂₀-Alkylengruppe steht,
- R₃ für ein Wasserstoffatom oder eine C₁-C₆-Alkylgruppe steht,
- R₄ für eine C₁-C₆-Alkylgruppe steht
- a, für eine ganze Zahl von 1 bis 3 steht, und
- b für die ganze Zahl 3 - a steht.

Die Substituenten R₁, R₂, R₃, R₄ und L in den Verbindungen der Formel (I) sind nachstehend beispielhaft erläutert:
Beispiele für eine C₁-C₆-Alkylgruppe sind die Gruppen Methyl, Ethyl, Propyl, Isopropyl, n-Butyl, s-Butyl und t-Butyl, n-Pentyl und n-Hexyl. Propyl, Ethyl und Methyl sind bevorzugte Alkylreste. Beispiele für eine C₂-C₆-Alkenylgruppe sind Vinyl, Allyl, But-2-enyl, But-3-enyl sowie Isobutenyl, bevorzugte C₂-C₆-Alkenylreste sind Vinyl und Allyl. Bevorzugte Beispiele für eine Hydroxy-C₁-C₆-alkylgruppe sind eine Hydroxymethyl-, eine 2-Hydroxyethyl-, eine 2-Hydroxypropyl, eine 3-Hydroxypropyl-, eine 4-Hydroxybutylgruppe, eine 5-Hydroxypentyl- und eine 6-Hydroxyhexylgruppe; eine 2-Hydroxyethylgruppe ist besonders bevorzugt. Beispiele für eine Amino-C₁-C₆-alkyl-gruppe sind die Aminomethylgruppe, die 2-Aminoethylgruppe, die 3-Aminopropylgruppe. Die 2-Aminoethylgruppe ist besonders bevorzugt. Beispiele für eine lineare zweiwertige C₁-C₂₀-Alkylengruppe sind beispielsweise die Methylen-gruppe (-CH₂-), die Ethylengruppe (-CH₂-CH₂-), die Propylengruppe (-CH₂-CH₂-CH₂-) und die Butylengruppe (-CH₂-CH₂-CH₂-CH₂-). Die Propylengruppe (-CH₂-CH₂-CH₂-) ist besonders bevorzugt. Ab einer Kettenlänge von 3 C-Atomen können zweiwertige Alkylengruppen auch verzweigt sein. Beispiele für verzweigte, zweiwertige C₃-C₂₀-Alkylengruppen sind (-CH₂-CH(CH₃)-) und (-CH₂-CH(CH₃)-CH₂-).

In den organischen Siliciumverbindungen der Formel (I)

R₁R₂N-L-Si(OR₃)ₐ(R₄)_{b} (I),

stehen die Reste R₁ und R₂ unabhängig voneinander für ein Wasserstoffatom oder eine C₁-C₆-Alkylgruppe. Ganz besonders bevorzugt stehen die Reste R₁ und R₂ beide für ein Wasserstoffatom.

Im Mittelteil der organischen Siliciumverbindung befindet sich die Struktureinheit oder der Linker -L- der für eine lineare oder verzweigte, zweiwertige C₁-C₂₀-Alkylengruppe steht.

Eine zweiwertige C₁-C₂₀-Alkylengruppe kann alternativ auch als eine divalente oder zweibindige C₁-C₂₀-Alkylengruppe bezeichnet werden, womit gemeint ist, dass jede Gruppierung L zwei Bindungen eingehen kann. Eine Bindung erfolgt von der Aminogruppe R1R2N zum Linker L, und die zweite Bindung besteht zwischen dem Linker L und dem Siliciumatom.

Bevorzugt steht -L- für eine lineare, zweiwertige (d.h. divalente) C₁-C₂₀-Alkylengruppe. Weiter bevorzugt steht -L- für eine lineare zweiwertige C₁-C₆-Alkylengruppe. Besonders bevorzugt steht -L- für eine Methylengruppe (-CH₂-), eine Ethylengruppe (-CH₂-CH₂-), eine Propylengruppe (-CH₂-CH₂-CH₂-) oder eine Butylengruppe (-CH₂-CH₂-CH₂-CH₂-). Ganz besonders bevorzugt steht L für eine Propylengruppe (-CH₂-CH₂-CH₂-).

Die lineare Propylengruppe (-CH₂-CH₂-CH₂-) kann alternativ auch als Propan-1,3-diylgruppe bezeichnet werden.

Die erfindungsgemäßen organischen Siliciumverbindungen der Formel (I)

R₁R₂N-L-Si(OR₃)ₐ(R₄)_{b} (I),

tragen jeweils ein einem Ende die Silicium-haltige Gruppierung -Si(OR₃)ₐ(R₄)_{b}.

In der endständigen Struktureinheit -Si(OR₃)ₐ(R₄)_{b} steht der Rest R₃ für ein Wasserstoffatom oder eine C₁-C₆-Alkylgruppe, und der Rest R₄ steht für eine C₁-C₆-Alkylgruppe. Besonders bevorzugt stehen R₃ und R₄ unabhängig voneinander für eine Methylgruppe oder eine Ethylgruppe.

Hierbei steht a für eine ganze Zahl von 1 bis 3, und b steht für die ganze Zahl 3 - a. Wenn a für die Zahl 3 steht, dann ist b gleich 0. Wenn a für die Zahl 2 steht, dann ist b gleich 1. Wenn a für die Zahl 1 steht, dann ist b gleich 2.

Besonders vorteilhafte Effektpigmente konnten erzeugt werden, wenn die organische Siliciumverbindung der Formel (I) entspricht, in welcher die Reste R₃, R₄ unabhängig voneinander für eine Methylgruppe oder für eine Ethylgruppe stehen.

Weiterhin konnten vorteilhafte Effektpigmente erhalten werden, wenn die organische Siliciumverbindung der Formel (I) entspricht, in welcher der Rest a für die Zahl 3 steht. In diesem Fall steht der Rest b für die Zahl 0.

Ganz besonders vorteilhafte Effektpigmente konnten erhalten werden, wenn die organische Siliciumverbindung der Formel (I) entspricht, wobei
- R₃, R₄ unabhängig voneinander für eine Methylgruppe oder für eine Ethylgruppe stehen und
- a für die Zahl 3 steht und
- b für die Zahl 0 steht.

Ebenfalls ganz besonders vorteilhafte Effektpigmente konnten erhalten werden, wenn die organische Siliciumverbindung der Formel (I) entspricht,

R₁R₂N-L-Si(OR₃)ₐ(R₄)_{b} (I),

wobei
- R₁, R₂ beide für ein Wasserstoffatom stehen, und
- L für eine lineare, zweiwertige C₁-C₆-Alkylengruppe, bevorzugt für eine Propylengruppe (-CH₂-CH₂-CH₂-) oder für eine Ethylengruppe (-CH₂-CH₂-), steht,
- R₃ für ein Wasserstoffatom, eine Ethylgruppe oder eine Methylgruppe steht,
- R₄ für eine Methylgruppe oder für eine Ethylgruppe steht,
- a für die Zahl 3 steht und
- b für die Zahl 0 steht.

Zur Lösung der erfindungsgemäßen Aufgabenstellung besonders gut geeignete organische Siliciumverbindungen der Formel (I) sind
- (3-Aminopropyl)triethoxysilan 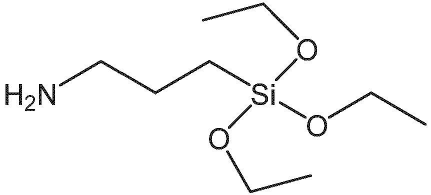
- (3-Aminopropyl)trimethoxysilan 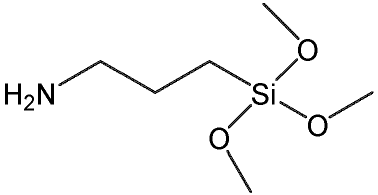
- 1-(3-Aminopropyl)silantriol 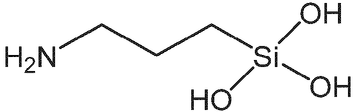
- (2-Aminoethyl)triethoxysilan 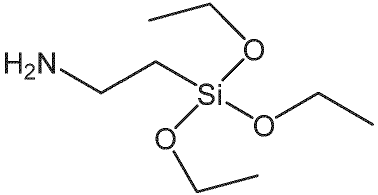
- (2-Aminoethyl)trimethoxysilan 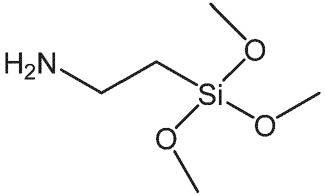
- 1-(2-Aminoethyl)silantriol 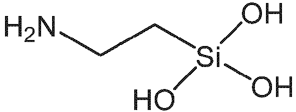
- (3-Dimethylaminopropyl)triethoxysilan 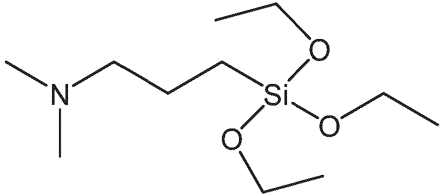
- (3-Dimethylaminopropyl)trimethoxysilan 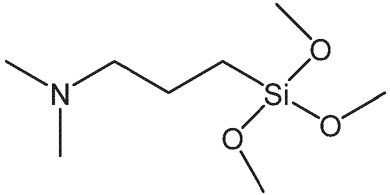
- 1-(3-Dimethylaminopropyl)silantriol 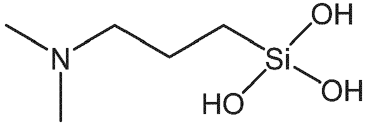
- (2-Dimethylaminoethyl)triethoxysilan. 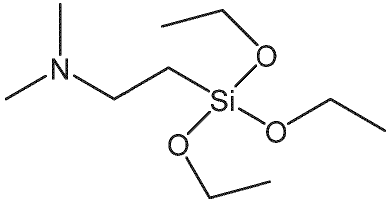
- (2-Dimethylaminoethyl)trimethoxysilan und 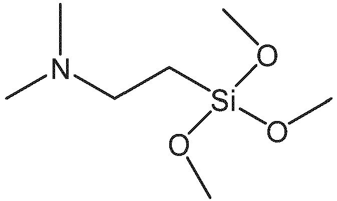
- 1-(2-Dimethylaminoethyl)silantriol

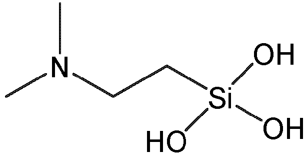

Die vorgenannten organischen Siliciumverbindungen der Formel (I) sind kommerziell erhältlich. (3-Aminopropyl)trimethoxysilan kann beispielsweise von Sigma-Aldrich käuflich erworben werden. Auch (3-Aminopropyl)triethoxysilan ist kommerziell bei der Firma Sigma-Aldrich erwerblich.

Bereits der Zusatz geringer Wassermengen führt bei organischen Siliciumverbindungen mit basischer Gruppe der Formel (I) zur Hydrolyse. Die Hydrolyseprodukte und/oder organischen Siliciumverbindungen mit einer basischen Gruppe der Formel (I) und mit mindestens einer Hydroxygruppe und/oder die Hydrolyseprodukte der ausgewählten Metallalkoxide können in einer Kondensationsreaktion miteinander reagieren. Aus diesem Grund können sowohl die siliciumorganischen Verbindungen basischer Gruppe der Formel (I) als auch deren Hydrolyse- und/oder Kondensationsprodukte sowie die Kondensationsprodukte mit den Hydrolyseprodukten der ausgewählten Metallalkoxide in der mindestens einen Schicht enthalten sein. Bei Verwendung von siliciumorganischen Verbindungen mit basischer Gruppe der Formel (I) können sowohl die organischen Siliciumverbindungen mit basischer Gruppe der Formel (I) als auch deren Kondensationsprodukte mit sich selbst und/oder mit den Hydrolyseprodukten der ausgewählten Metallalkoxide in der mindestens einen Schicht enthalten sein.

Unter einem Kondensationsprodukt wird entweder ein Produkt verstanden, dass durch Reaktion von mindestens zwei organischen Siliciumverbindungen mit jeweils mindestens einer Hydroxylgruppen oder hydrolysierbaren Gruppen pro Molekül unter Abspaltung von Wasser und/oder unter Abspaltung von einem Alkanol entsteht. Die Kondensationsprodukte können beispielsweise Dimere, aber auch Trimere oder Oligomere sein, wobei die Kondensationsprodukte mit den Monomeren im Gleichgewicht stehen. Abhängig von der eingesetzten oder in der Hydrolyse verbrauchten Wassermenge verschiebt sich das Gleichgewicht von monomerer organischen Siliciumverbindungen zu Kondensationsprodukt.

Unter einem Kondensationsprodukt wird auch ein Produkt verstanden, dass durch Reaktion von mindestens einer organischen Siliciumverbindung einer basischen Gruppe der Formel (I) mit einem Hydrolyseprodukt oder Kondensationsprodukt der ausgewählten Metallalkoxide unter Abspaltung von Wasser und/oder unter Abspaltung von einem Alkanol entsteht.

Durch Einsatz von Säuren und/oder Basen können die Hydrolyse und/oder die Kondensationsreaktion beeinflusst werden. So kann beispielsweise die Ausbildung der mindestens einen Schicht hinsichtlich Dicke, Kondensationsgrad der Kondensationsprodukte, Vernetzungsgrad der Kondensationsprodukte, Reaktionsgeschwindigkeit beeinflusst und gesteuert werden.

Das in dem nass-chemischen Beschichtungsverfahren eingesetzte Metallalkoxid ist ausgewählt aus der Gruppe bestehend aus Tetramethylorthosilikat, Tetraethylorthosilikat, Tetraisopropylorthosilikat und Mischungen daraus, wobei Tetraethylorthosilikat bevorzugt ist.

Die Schichten A und C dienen insbesondere auch als Korrosionsschutz als auch der chemischen und physikalischen Stabilisierung. Besonders bevorzugt enthalten die Schichten A und C Siliciumdioxid, das nach dem Sol-Gel-Verfahren aufgebracht wird.

Die mindestens eine Schicht kann ferner eine oder mehrere farbgebende Verbindungen aus der Gruppe der Pigmente und/oder der direktziehenden Farbstoffe enthalten.

Die Teilchengröße der eingesetzten farbgebende Verbindung richtet sich insbesondere danach, in welcher Schicht die farbgebende Schicht vorhanden ist. Die farbgebende Verbindung weist vorzugsweise eine Teilchengröße D₉₀ auf, welche kleiner als die Schichtdicke der mindestens einen Schicht ist. Mehr bevorzugt ist die Teilchengröße D₉₅ der farbgebenden Verbindung kleiner als die Schichtdicke der mindestens einen Schicht. Noch mehr bevorzugt ist die Teilchengröße D₉₉ der farbgebenden Verbindung kleiner als die Schichtdicke der mindestens einen Schicht. Ganz besonders bevorzugt ist die Teilchengröße D₁₀₀ der farbgebenden Verbindung kleiner als die Schichtdicke der mindestens einen Schicht. Die Teilchengröße der farbgebenden Verbindung kann beispielsweise unter Verwendung von dynamischer Lichtstreuung (DLS) oder statischer Lichtstreuung (SLS) bestimmt werden. D₉₀ bedeutet, dass 90% der Teilchen der farbgebenden Verbindung kleiner sind als die Schichtdicke der mindestens einen Schicht. Entsprechend bedeutet D₉₅, dass 95% der Teilchen der farbgebenden Verbindung kleiner sind als die Schichtdicke der mindestens einen Schicht, usw.

Die Menge an farbgebender Verbindung aus der Gruppe der Pigmente und/oder direktziehenden Farbstoffe in der mindestens einen Schicht beträgt vorzugsweise bis zu 5 Gew.-%, bezogen auf das Gesamtgewicht der mindestens einen Schicht.

Entsprechend kann es bevorzugt sein, dass die mindestens eine Schicht nass-chemisch unter Verwendung eines Metallalkoxids, einer siliciumorganischen Verbindung mit einer basischen Gruppe und einer farbgebenden Verbindung aus der Gruppe der Pigmente und/oder direktziehenden Farbstoffe hergestellt worden ist.

Ein weiterer Gegenstand der Anmeldung ein Verfahren zur Herstellung eines Effektpigments, umfassend a) ein Substratplättchen aus einem Metall oder aus einer Legierung und b) eine Beschichtung, umfassend die Schritte:
(α) Suspendieren des Substratplättchens in einem organischen oder wässrigen Lösungsmittel und
(β) Beschichten des in Schritt (α) suspendierten Substratplättchens im Rahmen eines Sol-Gel-Verfahrens unter Verwendung eines Metallalkoxids und einer siliciumorganischen Verbindung mit einer basischen Gruppe,
wobei das in dem Sol-Gel-Verfahren eingesetzte Metallalkoxid ausgewählt ist aus der Gruppe bestehend aus Tetramethylorthosilikat, Tetraethylorthosilikat, Tetraisopropylorthosilikat und Mischungen daraus.

Zusätzlich zu dem Tetraalkoxysilan können Alkyltrialkoxysilane in dem nass-chemischen Beschichtungsverfahren zur Herstellung der mindestens einen Schicht, beispielsweise der Schicht A oder C, eingesetzt werden.

Geeignete Alkyltrialkoxysilane umfassen beispielsweise Methyltrimethoxysilan, Methyltriethoxysilan, Ethyltrimethoxysilan, Ethyltriethoxysilan, Propyltrimethoxysilan, Propyltriethoxysilan, Hexyltrimethoxysilan, Hexyltriethoxysilan, Octyltrimethoxysilan, Octyltriethoxysilan, Dodecyltrimethoxysilan, Dodecyltriethoxysilan, Octadecyltrimethoxysilan und/oder Octadecyltriethoxysilan.

Die siliciumorganischen Verbindung mit einer basischen Gruppe ist vorzugsweise ausgewählt aus der Gruppe bestehend aus
- (3-Aminopropyl)triethoxysilan
- (3-Aminopropyl)trimethoxysilan
- -1-(3-Aminopropyl)silantriol
- (2-Aminoethyl)triethoxysilan
- (2-Aminoethyl)trimethoxysilan
- 1-(2-Aminoethyl)silantriol
- (3-Dimethylaminopropyl)triethoxysilan
- (3-Dimethylaminopropyl)trimethoxysilan
- -1-(3-Dimethylaminopropyl)silantriol
- (2-Dimethylaminoethyl)triethoxysilan
- (2-Dimethylaminoethyl)trimethoxysilan
- 1-(2-Dimethylaminoethyl)silantriol und
- Mischungen daraus.

Ganz besonders bevorzugt werden (3-Dimethylaminopropyl)triethoxysilan und/oder (3-Dimethylaminopropyl)trimethoxysilan als siliciumorganischen Verbindung mit einer basischen Gruppe eingesetzt.

Ein beispielhaftes Herstellverfahren umfasst das Dispergieren der unbeschichteten Substratplättchen oder der bereits mit Schicht A oder mit den Schichten A und B beschichteten Substratplättchen und der farbgebenden Verbindung aus der Gruppe der Pigmente in einer Lösung eines ausgewählten Metallalkoxids wie Tetraethylorthosilikat (üblicherweise in einer Lösung von organischem Lösungsmittel oder einer Mischung von organischem Lösungsmittel und Wasser mit mindestens 50 Gew.-% organisches Lösungsmittel wie ein C1 bis C4-Alkohol), und Zugabe einer schwachen Base oder Säure zur Hydrolysierung des ausgewählten Metallalkoxids, wodurch ein Film, umfassend das ausgewählte Metalloxid und die farbgebende Verbindung aus der Gruppe der Pigmente, auf der Oberfläche der (beschichteten) Substratplättchen entsteht.

Die Schicht B kann beispielsweise durch hydrolytische Zersetzung einer oder mehrerer organischer Metallverbindungen und/oder durch Fällung eines oder mehrerer gelöster Metallsalze sowie eine ggf. anschließende Nachbehandlung (zum Beispiel Überführen einer gebildeten hydroxidhaltigen Schichten in die Oxidschichten durch Tempern) hergestellt werden.

Die Effektpigmente auf Basis von beschichteten Substratplättchen weisen vorzugsweise eine Dicke von 70 bis 500 nm, besonders bevorzugt 100 bis 400 nm, insbesondere bevorzugt 150 bis 320 nm, beispielsweise 180 bis 290 nm, auf. Die geringe Dicke der beschichteten Substratplättchen wird insbesondere dadurch erzielt, dass die Dicke der unbeschichteten Substratplättchen gering ist, aber auch dadurch, dass die Dicken der Beschichtungen A und, falls vorhanden, C auf einen möglichst kleinen Wert eingestellt werden.

Die Haftung und Abriebbeständigkeit von Effektpigmenten auf Basis von Substratplättchen an/in einem Material, vorzugsweise keratinischen Material, kann deutlich erhöht werden, in dem die äußerste Schicht, je nach Aufbau Schicht A, B oder C, zusätzlich durch organische Verbindung wie Silane, Phosphorsäureester, Titanate, Borate oder Carbonsäuren modifiziert wird. Dabei sind die organischen Verbindungen an die Oberfläche der äußerstenSchicht A, B oder C gebunden. Die äußerste Schicht bezeichnet die Schicht, die räumlich am weitesten von dem Substratplättchen entfernt ist. Bei den organischen Verbindungen handelt es sich vorzugsweise um funktionelle Silanverbindungen, die an die Metalloxid-haltige Schicht A, B oder C binden können. Hierbei kann es sich entweder um mono- als auch um bifunktionelle Verbindungen handeln. Beispiele für bifunktionelle organische Verbindungen sind Methacryloxypropenyltrimethoxysilan, 3-Methacryloxypropyltrimethoxysilan, 3-Acryloxypropyltrimethoxysilan, 2-Acryloxyethyltrimethoxysilan, 3-Methacryloxypropyltriethoxysilan, 3-Acryloxypropyltrimethoxysilan, 2-Methacryloxyethyltriethoxysilan, 2-Acryloxyethyltriethoxysilan, 3-Methacryloxypropyltris(methoxyethoxy)silan, 3-Methacryloxypropyltris(butoxyethoxy)silan, 3-Methacryloxypropyltris(propoxy)silan, 3-Methacryloxypropyltris(butoxy)silan, 3-Acryloxypropyltris(methoxyethoxy)silan, 3-Acryloxypropyltris(butoxyethoxy)silan, 3-Acryloxypropyltris(butoxy)silan, Vinyltrimethoxysilan, Vinyltriethoxysilan, Vinylethyldichlorsilan, Vinylmethyldiacetoxysilan, Vinylmethyldichlorsilan, Vinylmethyldiethoxysilan, Vinyltriacetoxysilan, Vinyltrichlorsilan, Phenylvinyldiethoxysilan, oder Phenylallyldichlorsilan. Des Weiteren kann eine Modifizierung mit einem monofunktionellen Silan, insbesondere eines Alkylsilans oder Arylsilans, erfolgen. Dieses weist nur eine funktionelle Gruppe auf, welche kovalent an die Oberfläche des Effektpigments (d.h. an die äußerste Metalloxid-haltige Schicht) oder, bei nicht ganz vollständiger Bedeckung, an die Metalloberfläche anbinden kann. Der Kohlenwasserstoffrest des Silans weist vom Effektpigment weg. Je nach der Art und Beschaffenheit des Kohlenwasserstoffrests des Silans wird ein unterschiedlicher Grad der Hydrophobierung des Effektpigments erreicht. Beispiele für solche Silane sind Hexadecyltrimethoxysilan, Propyltrimethoxysilan, etc. Besonders bevorzugt sind Effektpigmente auf Basis von Siliciumdioxid-beschichteten Aluminiumsubstratplättchen mit einem monofunktionellen Silan oberflächenmodifiziert. Besonders bevorzugt sind Octyltrimethoxysilan, Octyltriethoxysilan, Hecadecyltrimethoxysilan sowie Hecadecyltriethoxysilan. Durch die veränderten Oberflächeneigenschaften / Hydrophobierung kann eine Verbesserung bezüglich Haftung, Abriebfestigkeit und Ausrichtung in der Anwendung erzielt werden.

### Beispiel

Zunächst wurden 200 g Al-Plättchen in Form von VMPs (Dicke zwischen 20 nm und 30 nm, d₅₀ = 12 µm) in Isopropanol suspendiert. Zu dieser Mischung wurden 45 g Tetraethoxysilan und 2 g (3-Aminopropyl)trimethoxysilan gegeben und die erhaltene Mischung wurde auf 60 °C erwärmt. Anschließend wurden 100 g Wasser und im Anschluss 6 g Ammoniak zugegeben und die erhaltene Mischung wurde für weitere 4 h gerührt. Danach wird die Mischung über eine Glasfritte abfiltriert und der erhaltene Filterkuchen bei 120 °C für 12 h getrocknet. Die erhaltene Schicht macht rund 40 Gew.-%, bezogen auf das Gesamtgewicht des Effektpigments, aus.

## Patentansprüche

1. Effektpigment, umfassend a) ein Substratplättchen aus einem Metall oder aus einer Legierung und b) eine Beschichtung,
wobei die Beschichtung mindestens eine Schicht aufweist, die nass-chemisch im Rahmen eines Sol-Gel-Verfahrens unter Verwendung eines Metallalkoxids und einer siliciumorganischen Verbindung mit einer basischen Gruppe hergestellt worden ist,
wobei das in dem Sol-Gel-Verfahren eingesetzte Metallalkoxid ausgewählt ist aus der Gruppe bestehend aus Tetramethylorthosilikat, Tetraethylorthosilikat, Tetraisopropylorthosilikat und Mischungen daraus, und
wobei die siliciumorganischen Verbindung mit einer basischen Gruppe die Formel (I) aufweist,
R₁R₂N-L-Si(OR₃)ₐ(R₄)_{b} (I),
wobei
- R₁, R₂ unabhängig voneinander für ein Wasserstoffatom oder eine C₁-C₆-Alkylgruppe stehen,
- L für eine lineare oder verzweigte, zweiwertige C₁-C₂₀-Alkylengruppe steht,
- R₃ für ein Wasserstoffatom oder eine C₁-C₆-Alkylgruppe steht,
- R₄ für eine C₁-C₆-Alkylgruppe steht
- a, für eine ganze Zahl von 1 bis 3 steht, und
- b für die ganze Zahl 3 - a steht.

2. Effektpigment gemäß Anspruch 1, **dadurch gekennzeichnet, dass** die Beschichtung das Substratplättchen vollständig umhüllt.

3. Effektpigment gemäß Anspruch 1 oder Anspruch 2, **dadurch gekennzeichnet, dass** die Beschichtung eine Schicht aufweist.

4. Effektpigment gemäß Anspruch 1 oder Anspruch 2, **dadurch gekennzeichnet, dass** die Beschichtung insgesamt mindestens zwei, vorzugsweise zwei oder drei, Schichten aufweist.

5. Effektpigment gemäß einem der Ansprüche 1 bis 4, **dadurch gekennzeichnet, dass** an der Beschichtung ferner eine monofunktionelle oder bifunktionelle organische Verbindung gebunden ist.

6. Effektpigment gemäß einem der Ansprüche 1 bis 5, **dadurch gekennzeichnet, dass** das Substratplättchen aus Aluminium ist.

7. Verfahren zur Herstellung eines Effektpigments, umfassend a) ein Substratplättchen aus einem Metall oder aus einer Legierung und b) eine Beschichtung, umfassend die Schritte:
(α) Suspendieren des Substratplättchens in einem organischen oder wässrigen Lösungsmittel,
(β) Beschichten des in Schritt (α) suspendierten Substratplättchens im Rahmen eines Sol-Gel-Verfahrens unter Verwendung eines Metallalkoxids und einer siliciumorganischen Verbindung mit einer basischen Gruppe,
wobei das in dem Sol-Gel-Verfahren eingesetzte Metallalkoxid ausgewählt ist aus der Gruppe bestehend aus Tetramethylorthosilikat, Tetraethylorthosilikat, Tetraisopropylorthosilikat und Mischungen daraus.

8. Verfahren gemäß Anspruch 7, **dadurch gekennzeichnet, dass** das in dem Sol-Gel-Verfahren eingesetzte Metallalkoxid Tetraethylorthosilikat ist.

9. Verfahren einem der Ansprüche 7 oder 8, **dadurch gekennzeichnet, dass** die siliciumorganischen Verbindung mit einer basischen Gruppe ausgewählt ist aus der Gruppe bestehend aus
- (3-Aminopropyl)triethoxysilan
- (3-Aminopropyl)trimethoxysilan
- -1-(3-Aminopropyl)silantriol
- (2-Aminoethyl)triethoxysilan
- (2-Aminoethyl)trimethoxysilan
- -1-(2-Aminoethyl)silantriol
- (3-Dimethylaminopropyl)triethoxysilan
- (3-Dimethylaminopropyl)trimethoxysilan
- -1-(3-Dimethylaminopropyl)silantriol
- (2-Dimethylaminoethyl)triethoxysilan.
- (2-Dimethylaminoethyl)trimethoxysilan
- 1-(2-Dimethylaminoethyl)silantriol und
- Mischungen daraus.

10. Verfahren gemäß einem der Ansprüche 7 bis 9, **dadurch gekennzeichnet, dass** in dem Sol-Gel-Verfahren ferner ein Alkyltrialkoxysilan eingesetzt wird.

## Claims

1. An effect pigment comprising a) a substrate platelet made of a metal or an alloy and b) a coating, wherein the coating comprises at least one layer which has been produced by a wet chemical process using a sol-gel method involving a metal alkoxide and an organosilicon compound containing a basic group,
wherein the metal alkoxide used in the sol-gel process is selected from the group consisting of tetramethyl orthosilicate, tetraethyl orthosilicate, tetraisopropyl orthosilicate and mixtures thereof, and wherein the organosilicon compound having a basic group has the formula (I),
R₁R₂N-L-Si(OR₃)ₐ(R₄)_{b} (I),
wherein
- R₁ and R₂ independently represent a hydrogen atom or a C₁-C₆ alkyl group,
- L represents a linear or branched, divalent C₁-C₂₀ alkylene group,
- R₃ represents a hydrogen atom or a C₁-C₆ alkyl group,
- R₄ represents a C₁-C₆ alkyl group
- a represents an integer from 1 to 3, and
- b represents the integer 3 - a.

2. Effect pigment according to claim 1, **characterised in that** the coating completely envelops the substrate platelet.

3. Effect pigment according to claim 1 or claim 2, **characterised in that** the coating comprises a single layer.

4. Effect pigment according to claim 1 or claim 2, **characterised in that** the coating comprises a total of at least two, preferably two or three, layers.

5. Effect pigment according to any one of claims 1 to 4, **characterised in that** a monofunctional or bifunctional organic compound is further bound to the coating.

6. Effect pigment according to any one of claims 1 to 5, **characterised in that** the substrate platelet is made of aluminum.

7. A method for producing an effect pigment comprising a) a substrate platelet made of a metal or an alloy and b) a coating, comprising the steps of:
(α) suspending the substrate platelet in an organic or aqueous solvent,
(β) coating the substrate platelet suspended in step (α) by a sol-gel process using a metal alkoxide and an organosilicon compound containing a basic group,
wherein the metal alkoxide used in the sol-gel process is selected from the group consisting of tetramethyl orthosilicate, tetraethyl orthosilicate, tetraisopropyl orthosilicate and mixtures thereof.

8. A method according to claim 7, **characterised in that** the metal alkoxide used in the sol-gel process is tetraethyl orthosilicate.

9. A method according to one of claims 7 or 8, **characterised in that** the organosilicon compound having a basic group is selected from the group consisting of
- (3-aminopropyl)triethoxysilane
- (3-aminopropyl)trimethoxysilane
- -1-(3-aminopropyl)silanetriol
- (2-aminoethyl)triethoxysilane
- (2-aminoethyl)trimethoxysilane
- -1-(2-aminoethyl)silane triol
- (3-dimethylaminopropyl)triethoxysilane
- (3-dimethylaminopropyl)trimethoxysilane
- -1-(3-dimethylaminopropyl)silane triol
- (2-Dimethylaminoethyl)triethoxysilane.
- (2-dimethylaminoethyl)trimethoxysilane
- 1-(2-dimethylaminoethyl)silane triol and
- mixtures thereof.

10. A method according to any one of claims 7 to 9, **characterised in that** an alkyltrialkoxysilane is also used in the sol-gel process.

## Revendications

1. Pigment à effet, comprenant a) une plaquette de substrat en métal ou en alliage et b) un revêtement,
ledit revêtement comportant au moins une couche qui a été préparée par voie chimique en phase humide dans le cadre d'un procédé sol-gel utilisant un alcoxyde métallique et un composé organosilicié comportant un groupe basique,
l'alcoxyde métallique utilisé dans le procédé sol-gel étant choisi dans le groupe constitué par l'orthosilicate de tétraméthyle, l'orthosilicate de tétraéthyle, l'orthosilicate de tétraisopropyle et leurs mélanges, et
le composé organosilicié comportant un groupe basique répondant à la formule (I),
R₁R₂N-L-Si(OR₃)ₐ(R₄)_{b} (I),
où
- R₁ et R₂ représentent, indépendamment l'un de l'autre, un atome d'hydrogène ou un groupe alkyle en C₁ à C₆,
- L représente un groupe alkylène divalent linéaire ou ramifié en C₁-C₂₀,
- R₃ représente un atome d'hydrogène ou un groupe alkyle en C₁-C₆,
- R₄ représente un groupe alkyle en C₁-C₆
- a représente un nombre entier compris entre 1 et 3, et
- b représente le nombre entier 3 - a.

2. Pigment à effet selon la revendication 1, **caractérisé en ce que** le revêtement enrobe entièrement la plaquette de substrat.

3. Pigment à effet selon la revendication 1 ou la revendication 2, **caractérisé en ce que** le revêtement comporte une couche.

4. Pigment à effet selon la revendication 1 ou la revendication 2, **caractérisé en ce que** le revêtement comporte au total au moins deux couches, de préférence deux ou trois.

5. Pigment à effet selon l'une des revendications 1 à 4, **caractérisé en ce qu'**un composé organique monofonctionnel ou bifonctionnel est en outre lié au revêtement.

6. Pigment à effet selon l'une des revendications 1 à 5, **caractérisé en ce que** la plaquette de substrat est en aluminium.

7. Procédé de fabrication d'un pigment à effet, comprenant a) une plaquette de substrat en métal ou en alliage et b) un revêtement, comprenant les étapes suivantes :
(α) la mise en suspension de la plaquette de substrat dans un solvant organique ou aqueux,
(β) Revêtement de la plaquette de substrat mise en suspension à l'étape (α) dans le cadre d'un procédé sol-gel, en utilisant un alcoxyde métallique et un composé organosilicié comportant un groupe basique,
l'alcoxyde métallique utilisé dans le procédé sol-gel étant choisi parmi le groupe constitué du tétraméthylorthosilicate, du tétraéthylorthosilicate, du tétraisopropylorthosilicate et de leurs mélanges.

8. Procédé selon la revendication 7, **caractérisé en ce que** l'alcoxyde métallique utilisé dans le procédé sol-gel est l'orthosilicate de tétraéthyle.

9. Procédé selon l'une des revendications 7 ou 8, **caractérisé en ce que** le composé organosilicié comportant un groupe basique est choisi parmi le groupe constitué de
- du (3-aminopropyl)triéthoxysilane
- le (3-aminopropyl)triméthoxysilane
- -1-(3-aminopropyl)silantriol
- (2-aminoéthyl)triéthoxysilane
- (2-aminoéthyl)triméthoxysilane
- -1-(2-aminoéthyl)silantriol
- (3-diméthylaminopropyl)triéthoxysilane
- (3-diméthylaminopropyl)triméthoxysilane
- -1-(3-diméthylaminopropyl)silantriol
- (2-diméthylaminoéthyl)triéthoxysilane.
- (2-diméthylaminoéthyl)triméthoxysilane
- 1-(2-diméthylaminoéthyl)silantriol et
- leurs mélanges.

10. Procédé selon l'une des revendications 7 à 9, **caractérisé en ce que**, dans le procédé sol-gel, on utilise en outre un alkyltrialcoxysilane.
